# FASCICULE DE BREVET EUROPEEN

(11) **EP 0 574 505 B1**
(45) Date de publication et mention de la délivrance du brevet: **17.12.1997**
(21) Numéro de dépôt: 92907773.3
(22) Date de dépôt: 09.03.1992
(51) Int. Cl.: A61L 2/20, A61L 9/015

(54) **PROCEDE DE DECONTAMINATION OU DE STERILISATION "IN SITU" D'UNE ENCEINTE ETANCHE SOUS VIDE ET DISPOSITIF POUR METTRE EN OEUVRE LE PROCEDE**
VERFAHREN ZUR "IN-SITU"-DEKONTAMINATION UND STERILISATION EINER VAKUUMKAMMER UND VORRICHTUNG ZUR DURCHFÜHRUNG DES VERFAHRENS
METHOD FOR DECONTAMINATING OR STERILIZING "IN SITU" A VACUUM SEALED CONTAINER AND DEVICE FOR IMPLEMENTING SUCH METHOD

(30) Priorité: 08.03.1991 FR 9102812
(43) Date de publication de la demande: 22.12.1993
(73) Titulaire: FONDATION NATIONALE DE TRANSFUSION SANGUINE, 75739 Paris Cédex 15 (FR)
(72) Inventeur: SCHMITTHAEUSLER, Roland, F-78180 Montigny-le-Bretonneux (FR); BARDAT, Annie, F-91470 Limours (FR)
(74) Mandataire: Warcoin, Jacques
(86) Numéro de dépôt international: FR9200212
(87) Numéro de publication internationale: WO9215337

(56) Documents cités:
- EP-A- 0 261 032
- EP-A- 0 286 506
- EP-A- 0 369 185
- WO-A-89/09068
- Paul Heinz List, Arzneiformenlehre, Wissenschaftliche Verlagsgesellschaft, Stuttgart, 1976, p. 378-380
- Pharmaceutical technology, Feb. 1996, Sterilization by Vapour Condensation

## Description

La présente invention concerne un procédé de décontamination ou de stérilisation "in situ" d'une enceinte étanche, notamment après un cycle de dessiccation sous vide d'un produit actif, par exemple lors d'une lyophilisation. Elle concerne également un dispositif pour mettre en oeuvre ledit procédé.

Parmi les procédés de conservation de produits labiles, la lyophilisation (sublimation de la glace sous pression réduite) constitue le moyen actuel le plus sûr de conserver à un produit son activité lors du stockage.

L'industrie pharmaceutique applique largement cette technique à tous les principes actifs susceptibles d'encourir une dénaturation lorsque la teneur en eau est trop importante. La lyophilisation est d'autant plus appropriée aux produits pharmaceutiques que cette technique s'applique en fin de production, au niveau du récipient final.

Si les procédés de préparation du lyophilisateur sont bien codifiés, notamment en ce qui concerne le nettoyage par les procédés NEP et la stérilisation à la vapeur, il n'en va pas de même pour le déchargement de la cuve de dessiccation. En effet, en cours de sublimation, des particules de produit actif ont pu être entraînées par le flux de vapeur d'eau et se retrouver de façon aléatoire dans l'atmosphère de la cuve. L'opérateur chargé de recueillir la fabrication est ainsi exposé au contact de ces aérosols, ce qui peut entraîner un risque important pour sa santé : absorption de principes actifs en doses non connues par voies d'introduction non validées : tractus respiratoire, zone oculaire, exposition cutanée.

Ces situations dangereuses sont particulièrement redoutables lorsque le produit lyophilisé contient des substances biologiques à potentiel réplicatif, telles que des bactéries, des virus, ou des acides nucléiques isolés. C'est le cas, par exemple, de préparations vaccinales lyophilisées ou de souches pathogènes de virus ou de bactéries destinées à la constitution de collections ou encore de vecteurs viraux ou bactériens préparés en vue d'une application vétérinaire ou agricole (parasitage ciblé d'agents nuisibles pour les animaux ou les plantes).

Le risque existe également lors de la lyophilisation de produits pouvant contenir des virus infectieux, comme par exemple HIV ou HBV, potentiellement présents dans le sang humain et ses dérivés, lorsque les contrôles de virémie ne sont pas assez sensibles ou donnent de faux négatifs ou ne sont pas fiables.

Les substances médicamenteuses lyophilisées présentent également un risque, dans la mesure où elles ont, à une concentration assez faible, une activité pharmacodynamique puissante : antibiotiques, anti-cancéreux, anti-inflammatoires stéroîdiens, immunodépresseurs, hormones.

Les risques sont très importants lorsque le récipient contenant ces substances est brisé à l'intérieur de l'enceinte du lyophilisateur, par suite d'un défaut de résistance toujours possible ou à l'occasion du bouchage automatique.

D'autre part, en déchargeant l'enceinte du lyophilisateur, on risque d'y introduire des bactéries, levures, moisissures provenant de l'atmosphère environnante.

L'utilisation de gaz de stérilisation tel que le formaldéhyde ou l'oxyde d'éthylène pose des problèmes délicats de dosage et de répartition à l'intérieur de l'enceinte d'un lyophilisateur.

Les germes stérilisés par ces moyens (vapeur, oxyde d'éthylène, formaldéhyde) ne sont plus capables de se multiplier, mais leurs éventuels résidus peuvent présenter un risque de toxicité : c'est le cas lors de la destruction de bactéries Gram négatif qui libèrent alors des lipopolysaccharides solubles, entraînant des réactions pyrogéniques dans les produits injectables.

Grâce à la présente invention, la Demanderesse propose de diminuer ces risques, soit par un nouveau moyen de décontamination "in situ" des produits nocifs libres ou libérés dans l'enceinte du lyophilisateur au cours du procédé de cryodessiccation lui-même, soit par un nouveau moyen de stérilisation de l'enceinte.

Dans le cadre de la présente description le terme "décontamination" sera employé pour designer l'utilisation d'un produit à activité biocide, dépyrogénante ou neutralisante en général.

La présente invention concerne un procédé de décontamination ou de stérilisation d'une enceinte étanche sous vide telle qu'un lyophilisateur dans laquelle se trouvent des microorganismes éventuellement pathogènes ou des substances dangereuses, notamment pyrogènes ou avant une influence néfaste sur l'environnement, choisies dans le groupe constitué par les virus, les bactéries (sporulées ou non), les champignons, les levures, les acides nucléiques. caractérisé en ce que lesdits microorganismes ou substances dangereuses sont déshydratés et en ce que l'on introduit sous vide dans l'enceinte un mélange décontaminant choisi dans le groupe constitué par :
- un mélange gazeux d'oxygène et d'ozone humidifié,
- un mélange gazeux de dioxyde de carbone, d'oxygène et d'ozone humidifié,
- un liquide aqueux choisi dans le groupe constitué par les solutions aqueuses d'acide péracétique, de peroxyde d'hydrogène ou d'alcools ou un mélange de celles-ci.

Selon une variante de l'invention le procédé est caractérisé en ce que un ou plusieurs récipients, fermés ou bien ouverts, contenant des microorganismes éventuellement pathogènes et/ou des substances dangereuses précitées se trouvent à l'intérieur de l'enceinte étanche et en ce que ledit gaz ou ledit liquide sont introduits avant l'ouverture de l'enceinte étanche pour prélever un ou les récipients.

Selon une autre variante de l'invention, l'enceinte du lyophilisateur peut être stérilisée avant chargement, par injection d'ozone, par exemple, pour assurer sa stérilité entre deux opérations.

Selon une des caractéristiques de l'invention, le microorganisme éventuellement pathogène et/ou le produit dangereux precité est déshydraté, en particulier par lyophilisation, atomisation ou séchage sous vide.

Le traitement de décontamination prend place de préférence après le cycle de lyophilisation que subit le produit. En effet, ce procédé permet de bénéficier du vide créé dans l'enceinte et de la possibilité de réfrigérer ou de chauffer les produits contenus dans l'enceinte. La température des mélanges décontaminants peut s'échelonner entre -50°C et +150°C. Lorsque le cycle de lyophilisation normal d'un produit à risque est terminé, les récipients de produit sec sont bouchés en automatique dans la cuve. L'enceinte est remise sous vide, si le bouchage a été effectué sous atmosphère inerte, ceci afin d'éviter les fuites vers l'extérieur et le système de refroidissement peut être maintenu en fonction, de façon à créer des surfaces de condensation. L'existence de cette dépression permet alors aisément la décontamination par vaporisation d'un liquide ou introduction d'un gaz, la réfrigération de l'enceinte permet la condensation sur les surfaces froides à décontaminer.

Le liquide destiné à être vaporisé doit bien entendu être adapté au microorganisme éventuellement pathogène à détruire ou au toxique à neutraliser.

On utilise notamment des solutions aqueuses d'acide peracétique, de péroxyde d'hydrogène ou d'alcool ou des mélanges, notamment d'acide peracétique et d'H202 ou d'alcool et d'H2O2.

L'injection d'un gaz préalablement généré fait également partie de l'invention, dans la mesure où ce gaz est doté notamment de propriétés oxydantes et virucides. Ces gaz, sont:
- les mélanges gazeux constitués d'oxygène et d'ozone humidifié ou d'oxygène, d'ozone et de dioxyde de carbone humidifié.

L'injection des phases liquides s'effectue à travers un injecteur disperseur qui provoque l'atomisation du mélange sous forme d'aérosol dans l'enceinte.

La pression à l'intérieur de l'enceinte est maintenue à une valeur suffisamment basse pour :
1/ rester en dépression par rapport à la pression atmosphérique extérieure, pour éviter les fuites d'agent inactivant.
2/ conserver une tension de vapeur suffisante pour obtenir une phase gazeuse du/des solvant/s injecté/s.

Le traitement dure de préférence de 30 minutes à 10 heures, selon la nature de la décontamination recherchée.

Pendant la durée du traitement, la modulation thermique de l'enceinte est ajustée selon l'effet recherché ; les températures négatives et les températures positives sont réglées en fonction de la stabilité du produit à décontaminer.

La méthode de décontamination selon l'invention s'applique à des produits biologiques potentiellement dangereux pouvant présenter un risque pour l'environnement s'ils ne sont pas confinés de façon hermétique et étanche dans un récipient : virus, bactéries (sporulées ou non), champignons, levures, acides nucléiques.

Dans le cas où le gaz est l'ozone, le procédé de stérilisation, de décontamination et de dépyrogénation est effectué dans des conditions définies de température et de pression auxquelles est soumis l'ozone.

L'ozone est généré en continu à partir de l'oxygène de l'air, mais pour des raisons de qualité et de conformité, la source de gaz est de l'oxygène pur, qui est introduit dans un générateur à haute tension. Le réglage du débit d'oxygène et la tension électrique appliquée déterminent la concentration d'ozone du gaz émergeant.

Ainsi, l'ozone n'est pas utilisé en gaz pur, mais généré à partir d'oxygène, de préférence à l'air, au moyen d'un générateur délivrant des concentrations connues d'ozone dans l'oxygène pouvant, par exemple, se trouver dans une fourchette comprise entre 4 µg/ml et 110 µg/ml (4 mg/l à 110 mg/l). Le mode de génération d'ozone à partir d'oxygène fait partie des connaissances techniques de l'homme de l'art : générateur à haute tension, à rayons ultraviolets, à cellule électrochimique, par exemple.

Le principe selon l'invention, consiste donc à injecter, à travers une vanne dans l'enceinte d'un lyophilisateur, un mélange d'oxygène/oxone humidifié. L'enceinte est préalablement placée sous vide (1 Pa à 0,1 Pa soit 10⁻² à 10⁻³ mbar). Le débit gazeux est assuré par la pression appliquée à la sortie du générateur.

Ce débit gazeux peut également être régulé par le dispositif d'injection gazeuse équipant le lyophilisateur, ce qui permet une addition contrôlée de gaz neuf constante.

L'enceinte peut être chargée en produits lyophilisés et la décontamination-stérilisation peut se placer en fin du cycle de lyophilisation.

Le mélange ozone/oxygène humidifié est alors injecté à la concentration voulue (4 µg/l à 110 mg/l) jusqu'à obtenir une pression dans l'enceinte inférieure ou égale à 0,8 bar (soit 8 x 10⁶ Pa).

La température des étagères peut être réglée entre - 20°C et 30°C.

Le temps de séjour du mélange ozone/oxygène est compris entre 4 minutes et 3 heures.

Le mode de stérilisation par l'ozone peut être amélioré dans certains cas de bactéries sporulées en humidifiant le gaz entre 10 % et 95 % de vapeur d'eau.

De même, le pH du mélange humidifié peut être réglé par addition de dioxyde de carbone, de façon à détruire plus rapidement des germes acido-sensibles.

Dans le cas d'un cycle de stérilisation c'est à dire avant introduction des agents pathogènes ou des substances dangereuses à traiter on peut opérer de la façon suivante :

Dans l'enceinte placée sous vide (1 Pa à 0,1 Pa, soit 10⁻² à 10⁻³ mbar), on injecte un mélange d'oxygène/ozone humidifié, contenant 30 à 70 mg d'ozone par litre de mélange, jusqu'à ce que la pression dans l'enceinte remonte à 0,8 bar (8 x 10⁶ Pa).

La température des étagères est réglée à + 10°C (± 20°C).

Le mélange est maintenu au contact de l'enceinte pendant une heure (± 30 min).

L'enceinte est ensuite remise sous vide à l'aide d'une pompe. Le trajet d'évacuation du gaz comporte un moyen de destruction de l'ozone résiduel, tel qu'un filtre à charbon actifs par exemple.

Une deuxième injection du mélange oxygène/ozone humidifié peut alors être effectuée dans des conditions semblables pour homogénéiser la distribution du gaz, dans le cas d'une enceinte à géométrie complexe.

Le temps de contact est maintenu pendant 30 minutes.

On peut selon un autre mode de réalisation de l'invention réguler l'injection d'ozone par le dispositif d'injection de gaz propre au lyophilisateur de façon à maintenir une pression partielle en ozone constante.

Le mélange oxygène/ozone est ensuite repompé et neutralisé.

La chambre remise sous vide peut être balayée par un gaz inerte stérile (azote par exemple) pour éliminer l'ozone résiduel.

Dans le cas d'un cycle de décontamination/dépyrogénation après que des produits viennent de subir la dessiccation on peut opérer de la façon suivante :

Dans l'enceinte placée sous vide (1 Pa à 0,1 Pa, soit 10⁻² à 10⁻³ mbar), on injecte un mélange d'oxygène/ozone humidifié, contenant 50 à 100 mg d'ozone par litre de mélange jusqu'à ce que la pression dans l'enceinte remonte à 0,8 bar (8 x 10⁶ Pa).

La température des étagères est réglée à + 10°C (± 20°C). Le temps de contact du mélange gazeux avec les contaminants provenant des aérosols de lyophilisation ou avec les substances pyrogènes générées est compris entre 30 minutes et 3 heures. Lorsque le contaminant est susceptible de contenir une double liaison carbone-carbone, il peut être utile d'accélérer l'hydrolyse de l'ozonide formé en injectant un gaz humidifié, contenant 10 à 95 % d'humidité relative.

A la fin du cycle, le mélange gazeux est aspiré au moyen d'une pompe à travers un dispositif d'élimination de l'ozone résiduel.

Ce dispositif est constitué d'une cartouche contenant du charbon actif. D'autres moyens d'élimination de l'ozone sont connus dans l'état de la technique : rayons UV, iodure de potassium, thermolyse, ...

L'enceinte peut alors être balayée par un gaz inerte (azote par exemple) pour éliminer les traces d'ozone résiduel.

L'invention s'applique également à des produits pharmacologiquement actifs pour la médecine humaine ou vétérinaire, notamment lorsqu'il est connu que le produit à traiter est sensible à l'oxydation.

C'est le cas de certaines classes de médicaments :
- Antibiotiques :
   . Oligomycine, comportant une double liaison C = C et un diène.
   . Pénicilline, comportant un noyau aromatique, ou une double liaison ou un atome de souffre dans la molécule.
- Immunodépresseurs :
   . Clyclosporine, comportant une double liaison.
- Anti-cancéreux :
   . Melphalan, comportant un noyau aromatique.
- Corticoïdes :
   . Dexamethasone, comportant une double liaison.
- Hormone : GHRH.
- Agents chélateurs :
   . Dimercaptol
- Prostaglandines.

Toutes ces substances sont capables de réagir avec l'ozone, puis éventuellement avec l'acide péracétique ou performique, pour donner des dérivés inactifs au plan pharmacologique.

L'invention s'applique également a l'élimination de composés indésirables pouvant accidentellement se trouver dans des produits actifs tels que le formol ou les endotoxines par exemple.

L'invention concerne également un dispositif pour mettre en oeuvre le procédé tel qu'il vient d'être décrit précédemment.

Ce dispositif d'injection et de contrôle des mélanges décontaminants permettant la mise en oeuvre du procédé est constitué d'une enceinte dans laquelle sont logés un ou plusieurs supports des produits à décontaminer, un ou plusieurs moyens de refroidissement et de chauffage et un moyen de mise sous vide caractérisé en ce que ladite enceinte est munie d'un ou plusieurs moyens d'approvisionnement en gaz ou liquide selon la description précédente et d'un ou plusieurs moyens d'évacuation desdits gaz ou liquides.

De préférence, le dispositif comporte un ou plusieurs moyens d'approvisionnement d'un gaz de balayage permettant d'accélérer l'évacuation desdits gaz ou liquides. Le dispositif comporte également un système de régulation de l'injection de gaz pour admettre des quantités contrôlées de gaz.

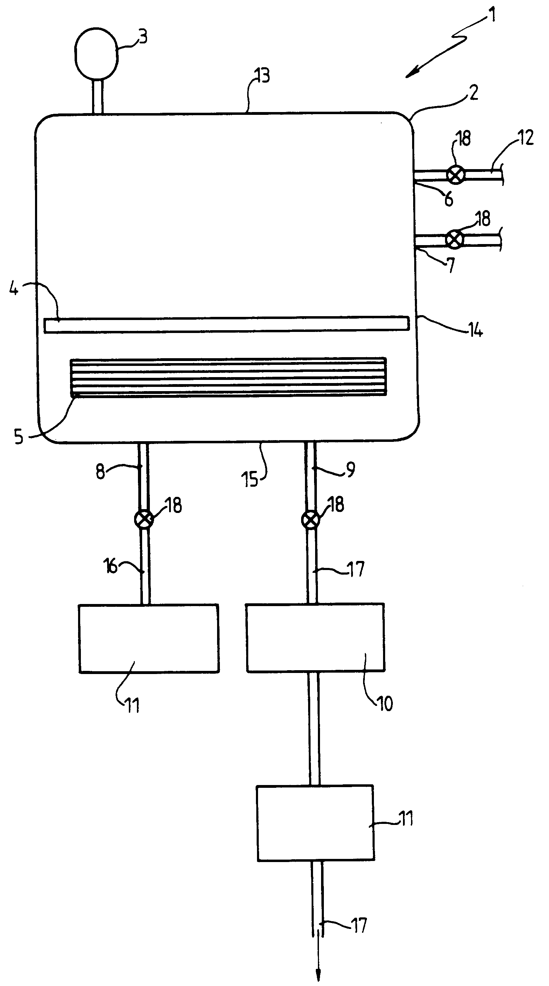

La figure unique en annexe est une vue en coupe schématisée d'un dispositif particulier permettant de mettre en oeuvre le procédé selon l'invention. Le dispositif 1 est constitué d'une enceinte 2, dans laquelle est logé un condenseur 5 surmonté à une distance appropriée d'un support 4 chauffant destiné à recevoir les récipients. A la paroi supérieure 13 de l'enceinte est fixée une jauge 3 de pression. Ladite enceinte est munie au niveau d'une paroi latérale 14, d'une entrée 6 reliée par une tubulure 12 à un générateur d'ozone (non figuré) et d'une entrée 7 pour un gaz inerte. La paroi inférieure 15 est munie d'une sortie 8 reliée par une tubulure 16 à une pompe à vide 11 et d'une autre sortie 9 reliée par un drain 17 à une pompe à vide 11, un piège 10 comportant du charbon actif étant monté en série entre l'entrée 9 et la pompe 11. Les tubulures ou drains précités sont munis de vannes de fermeture 18.

Les avantages et les applications possibles de la présente invention apparaîtront mieux dans les exemples suivants, donnés à titre d'illustration de l'invention, sans limiter son champ d'application.

### Exemple I

Une suspension de virus de la famille des bactériophages Microviridiae Ø X 174 est congelée et lyophilisée. La suspension contient au départ 10⁷ particules infectieuses par ml, exprimées en PFU/ml.

Le système de titration est effectué à l'aide d'une colonie uniforme d'E. Coli croissant sur gélose.

Par dilutions successives de 10 en 10 de la suspension virale, on obtient le titre de départ lorsque le nombre de plages de lyse apparues après incubation 18h à 37°C de la gélose ensemencée est compris entre 30 et 300.

Après lyophilisation, on injecte 500 ml d'alcool éthylique à 70% dans l'enceinte et on laisse en contact 30 minutes.

Les plateaux du lyophilisateur sont réglés à - 40°C.

### Résultats :

témoin lyophilisé bouché sous vide : 10⁶ PFU/ml
flacon ouvert en contact avec l'alcool 70% : 2,1 x 10² PFU/ml
Soit une réduction de 3,7 Log

### Exemple II

Dans les mêmes conditions que dans l'exemple I, on injecte du peroxyde d'hydrogène à 30 volumes à raison de 500 ml. On maintient en contact pendant 10 minutes.

La suspension virale témoin, bouchée sous vide, contenait après lyophilisation 10⁵ PFU/ml.

La flacon ouvert, en contact avec le peroxyde d'hydrogène, ne présente plus que 30 PFU/ml.

La réduction de titre viral obtenue est de 4,5 Log.

### Exemple III

Dans les mêmes conditions que dans l'exemple I, on injecte successivement après lyophilisation, d'abord 500 ml de peroxyde d'hydrogène à 30 volumes ; on laisse en contact 10 minutes ; puis on injecte 500 ml d'alcool éthylique 70%, qu'on laisse en contact pendant 30 minutes.

Dans cet exemple, la lyophilisation a été volontairement mal conduite, de façon à laisser une humidité résiduelle importante (> 20%) dans le produit traité.

Dans ce cas, la réduction de titre viral obtenue par le traitement n'est que de 1,1 Log (témoin bouché sous vide = 4 x 10⁵ PFU/ml; flacon en contact avec les agents inactivants = 3 x 10⁴ PFU/ml).

Cet exemple démontre que le produit à traiter doit être préalablement desséché, afin d'absorber en lieu et place d'eau, l'agent inactivant.

### Exemple IV

Dans les mêmes conditions que dans l'exemple I, on injecte successivement 500 ml de peroxyde d'hydrogène à 30 volumes et 500 ml d'alcool éthylique à 70%. Le flacon témoin lyophilisé contient 5 x 10³ PFU/ml.

Le flacon en contact avec les agents inactivants ne contient plus de particule infectieuse détectable. La réduction de titre viral obtenue est ≥ 3,7 Log.

### Exemple V

Une suspension de virus bactériophages Ø x 174 est lyophilisée dans son milieu de conservation comme dans l'exemple 1.

Après le cycle de dessiccation, on injecte dans la cuve du lyophilisateur une solution d'acide peracétique à 10% (vol/vol).

Le flacon témoin bouché sous vide contient 1,85 x 10⁴ PFU/ml.

Le flacon en contact avec l'agent inactivant ne contient aucune particule infectieuse détectable.

La réduction de titre viral est ≥ 4,3 Log.

### Exemple VI

Dans le but d'appliquer le procédé d'inactivation à un produit potentiellement infectieux, on a mélangé une suspension de virus bactériophages Ø x 176 à une solution d'immunoglobulines extraites de plasma humain : 100 ml de solution d'immunoglobulines humaines contiennent 20 ml de suspension de Ø x 174 à 1,52 x 10⁷ PFU/ml.

Après lyophilisation du produit, on injecte successivement dans la cuve du lyophilisateur 500 ml d'hydroxyde d'hydrogène à 30 volumes, puis après 10 minutes de contact, 500 ml d'alcool éthylique à 70%.

Le taux de réduction observé est de 1,52 Log.

### Exemple VII

Une solution d'immunoglobulines extraite du plasma humain est additionnée de virus bactériophage Ø x 174 comme dans l'exemple VI : 100 ml de solution d'immunoglobulines contiennent 20 ml de Ø x 174 à 3,7 x 10⁸ PFU/ml.

La solution finale contient encore 4,5 x 10⁴ PFU/ml ; la réduction de titre viral est attribuable à la présence d'anticorps spécifiques dans la préparation d'immunoglobulines utilisée.

Après lyophilisation, on injecte dans la cuve du lyophilisateur un mélange constitué d'alcool absolu 100%, dilué par du peroxyde d'hydrogène à 10 volumes, de telle façon que le titre alcoolique final soit de 70%.

Le flacon en contact avec l'agent inactivant ne contient aucune particule infectieuse détectable.

La réduction du titre viral est ≥ 4,65 Log.

### Exemple VIII

50 ml de solution d'albumine injectable extraite du plasma humain et contenant 50 g/l d'albumine est additionnée de 10 ml d'une suspension de virus bactériophage Ø x 174 titrant 6,55 x 10⁸ PFU/ml.

Après lyophilisation de la solution, on injecte dans l'enceinte un mélange gazeux contenant de l'oxygène et de l'ozone. L'ozone est obtenu par décharge électrique à haute tension dans un courant d'oxygène pur ; la quantité générée est de 40 µg/ml d'ozone. L'injection est effectuée jusqu'à obtenir une pression de - 0,2 bar dans l'enceinte. Le mélange est maintenu dans le lyophilisateur pendant 30 minutes.

Le gaz est ensuite aspiré à travers un agent neutralisant constitué d'une solution d'iodure de potassium.

La cuve est ensuite remise à pression atmosphérique. Aucun flacon traité dans le lyophilisateur ne contient de particules infectieuses détectables.

La réduction de titre viral obtenu est ≥ 8,8 Log.

### Exemple IX

Une solution d'immunoglobulines extraites de plasma humain s'est révélée non conforme lors des essais préconisés par la Pharmacopée, notamment en ce qui concerne la mesure de l'effet pyrogène sur lapins.

Ce produit non conforme lyophilisé est placé dans l'enceinte du lyophilisateur et mis sous vide.

On injecte alors le mélange alcool éthylène/peroxyde d'hydrogène tel qu'il est décrit dans l'exemple VII.

Après un temps de contact de 30 minutes, la cuve est purgée et les flacons traités sont bouchés sous vide.

L'essai des substances pyrogènes sur lapins montre une élévation de température de 1°45 pour 3 lapins à l'injection du produit non traité.

L'injection du produit traité selon les modalités de l'exemple X ne provoque plus qu'une élévation de température minime de 0,7°C pour 3 lapins.

Le produit traité est ainsi conforme aux prescriptions relatives à l'essai des substances pyrogènes.

### Exemple X

Une solution de facteur de la coagulation FVIII extraite du plasma humain a été accidentellement contaminée par des endotoxines d'origine bactérienne. Les flacons lyophilisés sont placés dans la cuve du lyophilisateur et évacues sous vide.

On injecte alors un mélange gazeux d'oxygène et d'ozone obtenu selon le même procédé que dans l'exemple VIII.

Le mélange est maintenu au contact du produit pendant 30 minutes.

Après remise à pression atmosphérique, les flacons sont titrés pour leur teneur en endotoxine par un test spécifique (Limulus Amebocyte Lysate : LAL). Les concentrations sont exprimées en Endotoxin Units/ml (EU/ml) par rapport à une endotoxine de référence.

Le flacon non traité contient 327 EU/ml ; le produit traité selon l'exemple X contient 116 EU/ml, soit une réduction de 64,5% du taux d'endotoxine.

### Exemple XI

Une suspension dans du bouillon nutritif de E. Coli contenant 10⁶ germes de E. Coli par ml est séchée sur bandelette de papier filtre.

La bandelette est ensuite replongée dans un bouillon nutritif pour démarrer la culture d'E. Coli.

Des dilutions successives dans NaCl 9 % (ou 9 g/l) sont inoculées sur plaques d'agar à la tryptone-soja pour numérer les germes résiduels.

Les bandelettes sont ensuite placées dans un lyophilisateur.

La stérilisation à l'ozone est effectuée selon le procédé décrit : 50 µg/ml 03, T° = 10°C, 30 min de contact.

Après remise en culture de la bandelette traitée selon ce procédé, aucune bactérie E. Coli n'est mise en évidence, après 7 jours de contact avec le milieu nutritif à 37°C.

L'essai en bandelette est repris avec une culture de bacillus subtilis, de façon analogue à l'essai précédent.

Dans les conditions de stérilisation à l'ozone : 50 µg/ml, T° = 10°C et 60 min de contact, aucune pousse de bacillus n'est mise en évidence, après 7 jours de contact de la bandelette traitée avec le milieu nutritif.

## Revendications

1. Procédé de décontamination ou de stérilisation d'une enceinte étanche sous vide telle qu'un lyophilisateur dans laquelle se trouvent des microorganismes éventuellement pathogènes ou des substances dangereuses, notamment pyrogènes ou avant une influence néfaste sur l'environnement, choisies dans le groupe constitué par les virus, les bactéries (sporulées ou non), les champignons, les levures, les acides nucléiques. caractérisé en ce que lesdits microorganismes ou substances dangereuses sont déshydratés et en ce que l'on introduit sous vide dans l'enceinte un mélange décontaminant choisi dans le groupe constitué par :
- un mélange gazeux d'oxygène et d'ozone humidifié,
- un mélange gazeux de dioxyde de carbone, d'oxygène et d'ozone humidifié,
- un liquide aqueux choisi dans le groupe constitué par les solutions aqueuses d'acide péracétique, de peroxyde d'hydrogène ou d'alcools ou un mélange de celles-ci.

2. Procédé selon la revendication 1, caractérisé en ce qu'un ou plusieurs récipients, fermés ou bien ouverts, contenant des microorganismes éventuellement pathogènes et/ou des substances dangereuses se trouvent à l'intérieur de l'enceinte étanche et en ce que ledit gaz ou ledit liquide sont introduits avant l'ouverture de l'enceinte étanche pour prélever un ou les récipients.

3. Procédé selon la revendication 1, caractérisé en ce que l'agent pathogène ou la substance dangereuse a été déshydraté par lyophilisation, atomisation ou séchage sous vide.

4. Procédé selon l'une des revendications 1 à 3, caractérisé en ce que le liquide susceptible de se vaporiser est de l'acide peracétique dans l'eau distillée.

5. Procédé selon l'une des revendications 1 à 8, caractérisé en ce que le liquide susceptible de se vaporiser est introduit dans l'enceinte par l'intermédiaire d'un injecteur/disperseur.

6. Procédé selon l'une des revendications 1 à 5, caractérisé en ce que l'enceinte étanche sous vide est une enceinte de lyophilisation.

7. Procédé selon l'une des revendications 1 à 6, caractérisé en ce que la pression à l'intérieur de l'enceinte est maintenue à une valeur suffisamment basse pour rester en dépression par rapport à la pression extérieure et pour conserver une tension de vapeur suffisante pour obtenir une phase gazeuse du liquide susceptible de se vaporiser ou du gaz.

8. Procédé selon l'une des revendications 1 à 7, caractérisé en ce que les mélanges décontaminants sont maintenus au contact des substances à décontamines de 30 minutes à 6 heures.

9. Procédé selon l'une des revendications 1 à 8 caractérisé en ce que les mélanges décontaminants peuvent être portés à des températures comprises entre - 50°C et + 150°C dans l'enceinte à traiter.

10. Dispositif (1) d'injection et de contrôle des mélanges décontaminants permettant la mise en oeuvre du procédé selon les revendications 1 à 9 constitué d'une enceinte (2) dans laquelle sont logés un ou plusieurs supports (4) des produits à décontaminer, un ou plusieurs moyens de chauffage (4), un ou plusieurs moyens de refroidissement (5) et un moyen (8) de mise sous vide, caractérisé en ce que ladite enceinte est munie d'un ou plusieurs moyens d'approvisionnement (6) en gaz ou liquide selon la revendication 1 et d'un ou plusieurs moyens d'évacuation desdits gaz ou liquides (9).

11. Dispositif selon la revendication 10, caractérisé en ce qu'il comporte un ou plusieurs moyens d'approvisionnement d'un gaz de balayage permettant d'accélérer l'évacuation desdits gaz ou liquides selon la revendication 1.

## Claims

1. Method for decontaminating or sterilizing a vacuum-sealed container such as a lyophilizer in which microorganisms, which may be pathogenic, or dangerous substances, especially pyrogenic substances or substances having a harmful influence on the environment, are present, the above being chosen from the group consisting of viruses, bacteria (sporulated or non-sporulated), fungi, yeasts and nucleic acids, characterized in that the said microorganisms or dangerous substances are dehydrated and in that a decontaminating mixture chosen from the group consisting of
- a gaseous mixture of oxygen and of wet ozone,
- a gaseous mixture of carbon dioxide, of oxygen and of wet ozone,
- an aqueous liquid chosen from the group consisting of aqueous solutions of peracetic acid, of hydrogen peroxide or of alcohols, or a mixture of these,
is introduced under vacuum into the container.

2. Method according to Claim 1, characterized in that one or more containers, closed or alternatively open, containing microorganisms which may be pathogenic and/or dangerous substances are present inside the sealed container and in that the said gas or the said liquid are introduced before opening the sealed container in order to remove a receptacle or the receptacles.

3. Method according to Claim 1, characterized in that the pathogenic agent or the dangerous substance was dehydrated by lyophilization, spray-drying or vacuum-drying.

4. Method according to one of Claims 1 to 3, characterized in that the liquid capable of vapourizing is peracetic acid in distilled water.

5. Method according to one of Claims 1 to 8, characterized in that the liquid capable of vapourizing is introduced into the container by means of an injector/disperser.

6. Method according to one of Claims 1 to 5, characterized in that the vacuum sealed container is a lyophilization container.

7. Method according to one of Claims 1 to 6, characterized in that the pressure inside the container is kept at a sufficiently low value in order to remain under vacuum relative to the external pressure and in order to preserve a vapour pressure sufficient to obtain a gaseous phase for the liquid capable of vapourizing or gas.

8. Method according to one of Claims 1 to 7, characterized in that the decontaminating mixtures are kept in contact with the substances to be decontaminated from 30 minutes to 6 hours.

9. Method according to one of Claims 1 to 8, characterized in that the decontamination mixtures may be heated to temperatures of between -50°C and +150°C in the container to be treated.

10. Device (1) for injecting and controlling decontaminating mixtures which permit the implementation of the method according to Claims 1 to 9 consisting of a container (2) in which are housed one or more supports (4) for the products to be decontaminated, one or more means of heating (4), one or more means of cooling (5) and a means (8) of placing under vacuum, characterized in that the said container is equipped with one or more means (6) of supplying gas or liquid according to Claim 1 and one or more means of evacuating the said gases or liquids (9).

11. Device according to Claim 10, characterized in that it comprises one or more means for supplying a flush gas which makes it possible to accelerate the evactuation of the said gases or liquids according to Claim 1.

## Patentansprüche

1. Verfahren zur Dekontamination oder Sterilisation eines dichten Behälters unter Vakuum (eines Vakuumbehälters), beispielsweise eines Lyophilisators, in dem sich befinden gegebenenfalls pathogene Mikroorganismen oder gefährliche, insbesondere pyrogene Substanzen oder solche, die einen schädigenden Einfluß auf die Umwelt haben, ausgewählt aus der Gruppe, die besteht aus Viren,(sporenbildenden oder nicht-sporenbildenden) Bakterien, Pilzen, Hefen und Nucleinsäuren, dadurch gekennzeichnet, daß die genannten Mikroorganismen oder gefährlichen Substanzen dehydratisiert werden und daß man in den Behälter unter Vakuum ein Dekontaminierungsmittelgemisch einführt, das ausgewählt wird aus der Gruppe, die besteht aus
- einem feuchten gasförmigen Gemisch aus Sauerstoff und Ozon,
- einem feuchten gasförmigen Gemisch aus Kohlendioxid, Sauerstoff und Ozon und
- einer wäßrigen Flüssigkeit, ausgewählt aus der Gruppe, die besteht aus wäßrigen Lösungen von Peressigsäure, Wasserstoffperoxid oder Alkoholen oder einer Mischung davon.

2. Verfahren nach Anspruch 1, dadurch gekennzeichnet, daß sich im Innern des Vakuumbehälters ein oder mehrere geschlossene oder auch offene Gefäße befinden, die gegebenenfalls pathogene Mikroorganismen und/oder gefährliche Substanzen enthalten, und daß das genannte Gas oder die genannte Flüssigkeit vor dem Öffnen des Vakuumbehälters eingeführt werden, um ein Gefäß oder die Gefäße zu entnehmen.

3. Verfahren nach Anspruch 1, dadurch gekennzeichnet, daß das pathogene Agens oder die gefährliche Substanz durch Lyophilisierung, Zerstäubung oder Trocknung unter Vakuum dehydratisiert worden ist.

4. Verfahren nach einem der Ansprüche 1 bis 3, dadurch gekennzeichnet, daß es sich bei der verdampfbaren Flüssigkeit um Peressigsäure in destilliertem Wasser handelt.

5. Verfahren nach einem der Ansprüche 1 bis 4, dadurch gekennzeichnet, daß die verdampfbare Flüssigkeit mittels eines Injektors/Dispergators in den Behälter eingeführt wird.

6. Verfahren nach einem der Ansprüche 1 bis 5, dadurch gekennzeichnet, daß der Vakuumbehälter ein Lyophilisierungs-Behälter ist.

7. Verfahren nach einem der Ansprüche 1 bis 6, dadurch gekennzeichnet, daß der Druck im Innern des Behälters bei einem ausreichend niedrigen Wert gehalten wird, um unterhalb des äußeren Druckes zu bleiben und einen Dampfdruck aufrechtzuerhalten, der ausreicht, um eine gasförmige Phase der verdampfbaren Flüssigkeit oder des Gases zu erhalten.

8. Verfahren nach einem der Ansprüche 1 bis 7, dadurch gekennzeichnet, daß die genannten Dekontaminierungsmittel-Gemische 30 min bis 6 h lang mit den zu dekontaminierenden Substanzen im Kontakt gehalten werden.

9. Verfahren nach einem der Ansprüche 1 bis 7, dadurch gekennzeichnet, daß die Dekontaminierungsmittel-Gemische in dem zu behandelnden Behälter auf Temperaturen zwischen -50 und +150°C gebracht werden können.

10. Vorrichtung (1) zur Injektion und Kontrolle (Steuerung) der Dekontaminierungsmittel-Gemische, welche die Durchführung des Verfahrens nach einem der Ansprüche 1 bis 9 ermöglicht, bestehend aus einem Behälter (2), in dem ein oder mehrere Träger (4) der zu dekontaminierenden Produkte angeordnet sind, einer oder mehreren Heiz-Einrichtungen (4), einer oder mehreren Kühl-Einrichtungen (5) und einer Evakuierungs(Abzugs)-Einrichtung (8), dadurch gekennzeichnet, daß der genannte Behälter mit einer oder mehreren Gas- oder Flüssigkeits-Beschickungs-Einrichtungen (6) nach Anspruch 1 und einer oder mehreren Evakuierungs(Abzugs)-Einrichtungen für die genannten Gase oder Flüssigkeiten (9) ausgestattet ist.

11. Vorrichtung nach Anspruch 10, dadurch gekennzeichnet, daß sie eine oder mehrere Spülgas-Einleitungs-Einrichtungen aufweist, welche die Beschleunigung der Evakuierung (des Abzugs) der genannten Gase oder Flüssigkeiten nach Anspruch 1 erlauben.
